# EUROPEAN PATENT SPECIFICATION

(11) **EP 1 415 646 B1**
(45) Date of publication and mention of the grant of the patent: **26.08.2009**
(21) Application number: 02257606.0
(22) Date of filing: 01.11.2002
(51) Int. Cl.: A61K 9/06, A61K 36/00, A61P 1/02

(54) **Gel comprising antimicrobial extract as an adjunct in the treatment of periodontitis**
Gel, einen antimikrobiellen Extrakt enthaltend als Zusatzmittel in der Therapie von Periodontitis
Gel comprenant un extrait antimicrobien comme auxiliaire en thérapie de la periodontite

(43) Date of publication of application: 06.05.2004
(73) Proprietor: Mahidol University, Nakornpathom 73170 (TH)
(72) Inventor: Rojanapanthu, Pleumchitt, Faculty of Pharmacy, Bangkok 10400 (TH); Gritsanapan, Wandee, Faculty of Pharmacy, Bangkok 10400 (TH); Sirirat, Mullika, Faculty of Pharmacy, Bangkok 10400 (TH); Amornchat, Cholticha, Faculty of Pharmacy, Bangkok 10400 (TH)
(74) Representative: Fairbairn, Angus Chisholm

(56) References cited:
- WO-A-92/09272
- GB-A- 2 317 339
- ANONYMOUS: "Faculty of Dentistry Mahidol University, Department of Microbiology, Academic Program" INTERNET ARTICLE, [Online] XP002235759 Retrieved from the Internet: <URL:http://www.dt.mahidol.ac.th/enghomepa ge/dep_microbio_academic.html> [retrieved on 2003-03-24] & RASSAMEEMASMAUNG S ET AL.: "Subgingival administration of Andrographis paniculata gel as an adjunct in the treatment of adult periodontitis" MAHIDOL JOURNAL, vol. 5, no. 1, 1998, pages 9-15, & ATSAWASUWAN P ET AL.: "Subgingival administration of Andrographis paniculata gel and Metronidasol gel as an adjunct in the treatment of adult periodontitis: Clinical and Microbiological effects" MAHIDOL JOURNAL, vol. 5, no. 2, 1998, pages 97-101,
- DATABASE WPI Section Ch, Week 200201 Derwent Publications Ltd., London, GB; Class B04, AN 2002-003044 XP002235761 & JP 2001 247469 A (LOTTE CO LTD), 11 September 2001 (2001-09-11)
- CHIOU WEN-FEI ET AL: "Mechanisms of suppression of inducible nitric oxide synthase (iNOS) expression in RAW 264.7 cells by andrographolide." BRITISH JOURNAL OF PHARMACOLOGY, vol. 129, no. 8, April 2000 (2000-04), pages 1553-1560, XP008015319 ISSN: 0007-1188
- RASSAMEEMASMAUNG S. ET AL: 'Subgingival administration of Andrographis paniculata gel as an adjunct in the treatment of adult periodontitis' MAHIDOL JOURNAL vol. 5, no. 1, 1998, BANGKOK, THAILAND, pages 9 - 15
- ATSAWASUWAN P. ET AL: 'Subgingival administration of Andrographis paniculata gel and Metronidasol gel as an adjunct in the treatment of adult periodontitis: clinical and microbiological effects' MAHIDOL JOURNAL vol. 5, no. 2, 1998, BANGKOK, THAILAND, pages 97 - 101

## Description

### FIELD OF THE INVENTION

This invention relates to the use of a plant extract to provide a product useful as an adjunct for the treatment of periodontitis. This product is provided in a sustained release dosage form with a biocompatible and biodegradable carrier. The product is in a form of herb extract gel that changes its structure to liquid crystal when contacted with gingival fluid (gum lymph) in the periodontal pocket. The product may be packed in the special syringe with a blunt, curved needle for convenient loading in the periodontal pocket.

### BACKGROUND OF THE INVENTION

At present it is reported that 90% of 40 year olds in the Thai population suffer from gingivitis, of which 80% can go on to develop to periodontitis when they are 60 years old. Patients have an inflammation of gingiva, alveolar bone, cementum and periodontal ligament, characterized by apical migration of the junctional epithelium, and loss of connective tissue attachment between the gingival fibre, periodontal ligament and cementum. There is resorption of the alveolar bone and development of pocket formation. If left untreated, the disease progresses to destruction of the supporting bone, whereby tooth mobility may be observed and sometimes periodontal abscess is found.

The basic treatment, from a patient's perspective, is oral hygiene instruction to control dental plaque and, from the dentist's perspective, is scaling and root planing. This procedure is not always sufficiently effective. This has led to the adjunctive use of antibiotics, usually in the form of a local delivery system rather than as a systemic antibiotic. Systemic antibiotics can cause side effects, for instance systemic metronidazole can irritate the gastro-intestinal tract, and, in repeated doses, can affect the nervous system, skin and kidney. Alcohol consumption is not permitted during the period of taking this drug. In the case of systemic tetracycline, this must be used long-term, and in a high dose in order to maintain an effective concentration in the periodontal pocket. Consequently, the normal microorganism environment may be disturbed and super infected by fungus, and development of bacterial resistance strains may occur.

An early development of a local delivery system used a non-biodegradable carrier substance that had to be removed after the release of the antibiotic agent. This system had many disadvantages, as follows: initially, it was difficult to insert the drug into the periodontal pocket, and after complete release of the antibiotic agent, the patient had to return to see the dentist for removal of the carrier. The need to remove the carrier might be harmful to gingival healing and also irritating to the gum.

Recently, controlled local delivery systems using biodegradable substances have been developed to solve this problem. These contain antibiotic and, although they are beneficial, they are expensive. Products of this type are commercially available as Elysol®, containing metronidazole, or as an ointment containing minocycline. Both of these products may be packed in a special syringe and needle, ready for loading into the periodontal pocket.

Studies of *Andrographis paniculata* gel in the treatment of periodontitis have been published by Rassameemasmaung S et al. in Mahidol Journal 5 (1998), pages 9-15 and by Atsawasuwan P et al. in Mahidol Journal 5 (1998), pages 97-101.

### DESCRIPTION OF THE INVENTION

According to the present invention, an antimicrobial extract is incorporated in a gel base to provide a gel, chip or ointment (hereinafter referred to as gel composition).

In a first aspect, a gel composition for adjunct treatment of periodontitis is provided, comprising:
(i) an antimicrobial extract having antimicrobial or antibacterial activity against periodontal pathogens, from one or more of the plants *Andrographis paniculata,* mangosteen *(Garcinia mangostana)* and turmeric *(Curcuma longa);*
(ii) a gel base containing a mixture of glyceryl monooleate and triglyceride, wherein the composition forms a liquid crystal structure on contacting gingival fluid.

In a second aspect, a process of preparing a composition for use as an adj unct in the treatment of periodontitis is provided, comprising:
(i) preparing an antimicrobial extract having antimicrobial or antibacterial activity against periodontal pathogens, from one or more of the plants *Andrographis paniculata,* mangosteen *(Garcinia mangostana)* and turmeric *(Curcuma longa);*
(ii) preparing a mixture of glyceryl monooleate and triglyceride as a gel base;
(iii) incorporating the antimicrobial extract into the gel base and mixing, to provide a composition that forms a liquid crystal structure on contacting gingival fluid.

The antimicrobial extract contains antimicrobial or antibacterial active compounds effective against periodontal pathogens, e.g. against the bacterium *Porphyromonas gingivalis* that is known to cause periodontitis. The extract is derived from *Andrographis paniculata,* a member of the plant family Acanthaceae, or from mangosteen *(Garcinia mangostana)* or turmeric *(Curcuma longa)* plants, which plants also contain antimicrobial or antibacterial active compounds effective against periodontal pathogens.

Preferably, the extract is derived from the plant *Andrographis paniculata.* Thus, the extract contains one or more of the following compounds: andrographolide (a diterpene lactone); deoxyandrographolide; 14-deoxy-11,12-didehydro-andrographolide; 14-deoxy-11-oxo-andrographolide; 14-deoxy-11-dehydro-andrographolide; andrographolide-19β-D glucoside; neoandrographolide; homo-andrographolide; andrographon; andrographan; andrographosterin; stigmasterol; peniculide.

The following procedure may suitably be used to prepare the antimicrobial extract:
Cleaned leaves and stem of *Andrographis paniculata* (Burmf.) Nees. are chopped into small pieces, dried in a hot air oven at 40-80°C for 2-8 hours, then powdered and passed through a sieve with 1-4 mm pore size, and kept in a well sealed bottle.
The powder is extracted with organic solvents e.g. alcohol, acetone, chloroform, or other solvents, in a proportion of 400-700 grams of powders: 3,000-7,000 grams of solvent, with a Soxhlet apparatus at 30-95°C for 3-80 hours.
Activated charcoal is added into the solvent extract (1-5 gram activated charcoal: 2-10 gram *Andrographis paniculata* extract), mixed and left for 1-5 hours in order to decolorize chlorophyll.
The mixture is filtered through a filter paper (Whatman no. 1), and the filtrate is concentrated by a rotary evaporator under reduced pressure at 30-95°C. The concentrated solution is then evaporated on a boiling water bath until a constant weight is attained, to provide the antimicrobial extract.

It will be appreciated that other suitable extraction methods, known to those skilled in the art, may instead be used in accordance with the invention, in order to prepare the antimicrobial extract. Leaves or other parts of the plants such as roots or rhizomes may be used, as appropriate, as raw material for extraction of the active ingredients. For *Andrographis paniculata* extract, preferably the leaves are used.

The gel base is prepared from a mixture of glyceryl monooleate and triglyceride. Glyceryl monooleate is commercially available, and may be prepared by reaction of oleic acid and glycerine in the presence of a suitable catalyst, to provide the monoglyceride ester of oleic acid and small amounts of other fatty acids. Thus, glyceryl monooleate is a mixture of the glyceride of oleic acid and other fatty acids, consisting mainly of the monoglyceride ester of oleic acid, with emollient property. Preferably, the glyceryl monooleate comprises at least 95 wt% monoglyceride ester of oleic acid.

Triglycerides include oils and fats, and are the main constituent of ordinary fat in foods. Triglycerides contain fatty acids, in the form of saturated fatty acids and/or unsaturated fatty acids, linked to a glycerol molecule.

The triglyceride used according to the present invention preferably is an oil, in particular a vegetable oil, for example, sesame oil, sunflower oil, soybean oil or safflower oil, or any mixture thereof.

The ratio of glyceryl monooleate : triglyceride is in the range from 2:1 to 10:1 by weight. The gel base mixture of glyceryl monooleate and triglyceride is incorporated in an amount of 60%-95%, by weight of the composition.

When glyceryl monooleate is contacted with water or the gingival exudates (gum lymph), it increases in viscosity and has a cubic mesophase structure. In combination with triglyceride, the cubic phase structure changes to hexagonal with the structure of liquid crystal. The liquid crystal structure can retain the active ingredient or the herb extract between the crystals, and releases the active ingredient content gradually, thus providing a sustained release dosage form.

The antimicrobial extract, preferably *Andrographis paniculata* extract, is incorporated, in an amount of 1-20%, preferably 1%-15%, by weight of the composition (% based on dry extract). The concentration of extract used must, however, still be suitable for the gel base to be able to change to a liquid crystal structure.

An antioxidant or preservative agent is preferably incorporated in the composition, in a preferred amount of 0.5%-5%, by weight of the composition. A preferred antioxidant is D-α-tocopherol acetate.

Small amounts of auxiliary substances conventionally used in gel formulations may also be included, for example stabilizers, homogenising agents, texturising agents, soothing agents. For example, glycerin may assist in providing a homogeneous texture to the gel that is also soothing to the wound. Collagen may assist in healing of the wound. Preferably, glycerin or collagen, or both, are incorporated in the composition, preferably in a total amount of 0.1-5%, by weight of the composition.

The obtained gel is preferably packed in a syringe with the curved, blunt needle suitable for loading the gel into the periodontal pocket. Other convenient packaging can be used.

### EXAMPLES

### Example 1

### 1. Extraction of Andrographis paniculata plant:

1.1 Cleaned leaves and stem of *Andrographis paniculata* (Burmf.) Nees. were chopped into small pieces, dried in a hot air oven at 60°C for 5 hours, then powdered and passed through a sieve with 3 mm pore size, and kept in a well sealed bottle.
1.2 The powders were extracted with alcohol as solvent, in a proportion of 550 grams of powders: 5,000 grams of solvent, with a Soxhlet apparatus at 65°C for 40 hours.
1.3 Activated charcoal was added into the solvent extract (3 gram activated charcoal : 6 gram *Andrographis paniculata* extract), mixed and left for 3 hours for decolorizing of chlorophyll.
1.4 The mixture was filtered through a filter paper (Whatman no. 1), and the filtrate was is concentrated by a rotary evaporator under reduced pressure at 65°C. The concentrated solution was then evaporated on a boiling water bath until a constant weight was attained.

### 2. Preparation of compositions for adjunct in the treatment of periodontitis:

### 2.1 Preparation of gel base:

Glyceryl monooleate, containing the glyceride of oleic acid and other fatty acids, and triglyceride (sesame oil) were mixed in a ratio of glyceryl monooleate : triglyceride of 6:1, by weight.

### 2.2 Incorporation of Andrographis paniculata extract into the gel:

To the gel base mixture were added D-α-tocopherol acetate, as antioxidant; glycerin; and *Andrographis paniculata* extract, and mixed thoroughly to provide a gel composition containing 76.5% glyceryl monooleate/triglyceride gel base mixture, 20% *Andrographis paniculata* extract, 2.5% D-α-tocopherol acetate, and 1% glycerin, by weight of the composition.

### 3. Clinical comparison studies:

In a clinical comparison study with Elysol® (metronidazole gel), the results show that the percentage of black pigmented colonies (anaerobic microorganisms that cause periodontitis) had significantly reduced in the case of using the *Andrographis paniculata* gel but no reduction was found in the case of using the metronidazole gel.

In a clinical comparison study with minocycline ointment, the results showed that the *Andrographis paniculata* gel significantly reduced the percentage of motile rod shaped microorganisms compared with groups treated with the minocycline ointment and control groups.

The gel was found to have a liquid crystal structure after being contacted with gingival fluid.

### Example 2

Using the same procedure and amounts as described in Example 1, a mangosteen extract was prepared by alcoholic extraction, and was incorporated into a gel base mixture of glyceryl monooleate and triglyceride (sesame oil) in a ratio of glyceryl monooleate : triglyceride of 6 : 1, by weight, together with D-α-tocopherol acetate as antioxidant, and glycerin. The mangosteen gel composition contained 76.5% glyceryl monooleate/triglyceride gel base mixture, 20% mangosteen extract, 2.5% D-α-tocopherol acetate, and 1% glycerin, by weight of the composition.

The gel formed a liquid crystal structure when contacted with water. Preliminary tests showed that the mangosteen gel was effective in reducing the percentage of microorganisms that cause periodontitis compared with control groups.

### Example 3

Using the same procedure and amounts as described in Example 1, a turmeric extract was prepared by alcoholic extraction, and was incorporated into a gel base mixture of glyceryl monooleate and triglyceride (sesame oil) in a ratio of glyceryl monooleate : triglyceride of 6 : 1, by weight, together with D-α-tocopherol acetate as antioxidant, and glycerin. The turmeric gel composition contained 76.5% glyceryl monooleate/triglyceride gel base mixture, 20% turmeric extract, 2.5% D-α-tocopherol acetate, and 1% glycerin, by weight of the composition.

The gel formed a liquid crystal structure when contacted with water. Preliminary tests showed that the turmeric gel was effective in reducing the percentage of microorganisms that cause periodontitis compared with control groups.

## Claims

1. A gel composition for adjunct treatment of periodontitis, comprising:
(i) an antimicrobial extract having antimicrobial or antibacterial activity against periodontal pathogens, from one or more of the plants *Andrographis paniculata,* mangosteen (*Garcinia mangostana*) and turmeric (*Curcuma longa*), in an amount of 1%-20% by weight of the composition;
(ii) a gel base containing a mixture of glyceryl monooleate and triglyceride wherein the weight ratio of glyceryl monooleate : triglyceride is in the range of 2:1 to 10:1, in an amount of 60%-95% by weight of the composition,
wherein the composition forms a liquid crystal structure on contacting gingival fluid.

2. A composition according to claim 1, comprising the antimicrobial extract in an amount of 1%-15% by weight of the composition.

3. A composition according to claim 1 or claim 2, wherein the composition is a sustained release gel, chip or ointment that is biodegradable.

4. A composition according to any preceding claim, wherein the triglyceride is one or more vegetable oils selected from sesame oil, sunflower oil, soybean oil and safflower oil.

5. A composition according to any preceding claim, wherein the antimicrobial extract is an extract from the plant *Andrographis paniculata*.

6. A composition according to any preceding claim, further comprising an antioxidant or preservative agent.

7. A composition according to claim 6, comprising D-α-tocopherol acetate in an amount of 0.5%-5% by weight of the composition, as an antioxidant.

8. A composition according to any preceding claim, further comprising glycerin or collagen, or both.

9. A composition according to claim 8, comprising glycerin in an amount of 0.1%-5% by weight of the composition.

10. A process of preparing a composition for use as an adjunct in the treatment of periodontitis, comprising:
(i) preparing an antimicrobial extract having antimicrobial or antibacterial activity against periodontal pathogens, from one or more of the plants *Andrographis paniculata*, mangosteen (*Garcinia mangostana*) and turmeric (*Curcuma longa*);
(ii) preparing a mixture of glyceryl monooleate and triglyceride as a gel base, wherein the weight ratio of glyceryl monooleate : triglyceride is in the range of 2:1 to 10:1;
(iii) incorporating the antimicrobial extract into the gel base, wherein the antimicrobial extract is incorporated in an amount of 1%-20% by weight of the composition and the gel base is incorporated in an amount of 60%-95% by weight of the composition, and mixing, to provide a composition that forms a liquid crystal structure on contacting gingival fluid.

11. A process according to claim 10, wherein the antimicrobial extract is incorporated in an amount of 1%-15% by weight of the composition.

12. A process according to claim 10 or claim 11, wherein the composition is prepared as a sustained release gel, chip or ointment that is biodegradable.

13. A process according to any of claims 10 to 12, wherein the triglyceride is one or more vegetable oils selected from sesame oil, sunflower oil, soyabean oil and safflower oil.

14. A process according to any of claims 10 to 13, wherein the antimicrobial extract is prepared from the plant *Andrographis paniculata*.

15. A process according to any of claims 10 to 14, further comprising incorporating D-α-tocopherol acetate in an amount of 0.5%-5% by weight of the composition, as an antioxidant.

16. A process according to any of claims 10 to 15, further comprising incorporating glycerin or collagen, or both, in a total amount of 0.1%-5% by weight of the composition.

17. The use of a composition according to any of claims 1 to 9 for the manufacture of an adjunct for the treatment of periodontitis.

## Patentansprüche

1. Gelzusammensetzung zur Zusatzbehandlung von Periodontitis, die aufweist:
(i) einen antimikrobiellen Extrakt mit antimikrobieller und antibakterieller Wirkung gegen periodontale Pathogene aus einer oder mehreren der Pflanzen *Andrographis paniculata*, Mangostan (*Garcinia mangostana*) und Kurkuma (*Curcuma longa*) in einem Anteil von 1-20 Gew.-% der Zusammensetzung;
(ii) eine Gelbasis, die ein Gemisch aus Glycerylmonooleat und Triglycerid, wobei das Gewichtsverhältnis von Glycerylmonooleat : Triglycerid im Bereich von 2:1 bis 10:1 liegt, in einem Anteil von 60-95 Gew.-% der Zusammensetzung enthält,
wobei die Zusammensetzung bei Kontakt mit Gingivaflüssigkeit eine Flüssigkristallstruktur bildet.

2. Zusammensetzung nach Anspruch 1, die einen antimikrobiellen Extrakt in einem Anteil von 1-15 Gew.-% der Zusammensetzung enthält.

3. Zusammensetzung nach Anspruch 1 oder Anspruch 2, wobei die Zusammensetzung ein Gel, Chip oder eine Salbe mit Depotwirkung ist, die biologisch abbaubar ist.

4. Zusammensetzung nach einem der vorstehenden Ansprüche, wobei das Triglycerid aus einem oder mehreren Pflanzenölen besteht, die unter Sesamöl, Sonnenblumenöl, Sojaöl und Färberdistelöl ausgewählt sind.

5. Zusammensetzung nach einem der vorstehenden Ansprüche, wobei der antimikrobielle Extrakt ein Extrakt aus der Pflanze *Andrographis paniculata* ist.

6. Zusammensetzung nach einem der vorstehenden Ansprüche, die ferner ein Antioxidationsmittel oder Konservierungsmittel aufweist.

7. Zusammensetzung nach Anspruch 6, die D-α-Tocopherolacetat in einem Anteil von 0,5-5 Gew.-% der Zusammensetzung als Antioxidationsmittel aufweist.

8. Zusammensetzung nach einem der vorstehenden Ansprüche, die ferner Glycerin oder Kollagen oder beide aufweist.

9. Zusammensetzung nach Anspruch 8, die Glycerin in einem Anteil von 0,1-5 Gew.-% der Zusammensetzung aufweist.

10. Verfahren zur Herstellung einer Zusammensetzung zur Verwendung als Zusatzstoff bei der Behandlung von Periodontitis, wobei das Verfahren aufweist:
(i) Herstellen eines antimikrobiellen Extrakts mit antimikrobieller oder antibakterieller Wirkung gegen periodontale Pathogene aus einer oder mehreren der Pflanzen *Andrographis paniculata*, Mangostan (*Garcinia mangostana*) und Kurkuma (*Curcuma longa*);
(ii) Herstellen eines Gemischs von Glycerylmonooleat und Triglycerid als Gelbasis, wobei das Gewichtsverhältnis von Glycerylmonooleat : Triglycerid im Bereich von 2:1 bis 10:1 liegt;
(iii) Zudosieren des antimikrobiellen Extrakts zu der Gelbasis, wobei der antimikrobielle Extrakt in einem Anteil von 1-20 Gew.-% der Zusammensetzung zudosiert wird und die Gelbasis in einem Anteil von 60-95 Gew.-% der Zusammensetzung zudosiert wird, und Vermischen, um eine Zusammensetzung bereitzustellen, die bei Kontakt mit Gingivaflüssigkeit eine Flüssigkristallstruktur bildet.

11. Verfahren nach Anspruch 10, wobei der antimikrobielle Extrakt in einem Anteil von 1-15 Gew.-% der Zusammensetzung zudosiert wird.

12. Verfahren nach Anspruch 10 oder Anspruch 11, wobei die Zusammensetzung als Gel, Chip oder Salbe mit Depotwirkung hergestellt wird, die biologisch abbaubar ist.

13. Verfahren nach einem der Ansprüche 10 bis 12, wobei das Triglycerid aus einem oder mehreren Pflanzenölen besteht, die unter Sesamöl, Sonnenblumenöl, Sojaöl und Färberdistelöl ausgewählt sind.

14. Verfahren nach einem der Ansprüche 10 bis 13, wobei der antimikrobielle Extrakt aus der Pflanze *Andrographis paniculata* hergestellt wird.

15. Verfahren nach einem der Ansprüche 10 bis 14, das ferner das Zudosieren von D-α-Tocopherolacetat in einem Anteil von 0,5-5 Gew.-% der Zusammensetzung als Antioxidationsmittel aufweist.

16. Verfahren nach einem der Ansprüche 10 bis 15, das ferner das Zudosieren von Glycerin oder Kollagen oder beiden in einem Gesamtanteil von 0,1-5 Gew.-% der Zusammensetzung aufweist.

17. Verwendung der Zusammensetzung nach einem der Ansprüche 1 bis 9 für die Herstellung eines Zusatzstoffs zur Behandlung von Periodontitis.

## Revendications

1. Composition en gel pour le traitement d'appoint de la parodontite, comprenant :
(i) un extrait antimicrobien ayant une activité antimicrobienne ou antibactérienne contre les pathogènes parondontaux, issu d'une ou plusieurs plantes parmi *Andrographis paniculata*, le mangoustan (*Garcinia mangostana*), et le curcuma (*Curcuma longa*), dans une quantité de 1% à 20% en poids de la composition;
(ii) une base de gel contenant un mélange de monooléate de glycéryle et de triglycéride dans lequel le rapport pondéral monooléate de glycéryle:triglycéride est compris entre 2:1 et 10:1, dans une quantité de 60% à 95% en poids de la composition,
la composition formant une structure cristalline liquide au contact du fluide gingival.

2. Composition selon la revendication 1, comprenant l'extrait antimicrobien dans une quantité de 1% à 5% en poids de la composition.

3. Composition selon la revendication 1 ou la revendication 2, laquelle composition est un gel, une puce ou une pommade à libération prolongée qui est biodégradable.

4. Composition selon l'une quelconque des revendications précédentes, dans laquelle le triglycéride consiste en une ou plusieurs huiles végétales choisies parmi l'huile de sésame, l'huile de tournesol, l'huile de soja et l'huile de carthame.

5. Composition selon l'une quelconque des revendications précédentes, dans laquelle l'extrait antimicrobien est un extrait de la plante *Andrographis paniculata*.

6. Composition selon l'une quelconque des revendications précédentes, comprenant en outre un antioxydant ou un agent conservateur.

7. Composition selon la revendication 6, comprenant de l'acétate de D-α-tocophérol dans une quantité de 0,5% à 5% en poids de la composition, en tant qu'antioxydant.

8. Composition selon l'une quelconque des revendications précédentes, comprenant en outre de la glycérine ou du collagène, ou les deux.

9. Composition selon la revendication 8, comprenant de la glycérine dans une quantité de 0,1% à 5% en poids de la composition.

10. Procédé de préparation d'une composition à utiliser en appoint dans le traitement de la parodontite, consistant à :
(i) préparer un extrait antimicrobien ayant une activité antimicrobienne ou antibactérienne contre les pathogènes parondontaux, à partir d'une ou plusieurs plantes parmi *Andrographis paniculata*, le mangoustan (*Garcinia mangostana*), et le curcuma (*Curcuma longa*);
(ii) préparer un mélange de monooléate de glycéryle et de triglycéride en tant que base de gel, où le rapport pondéral monooléate de glycéryle:triglycéride est compris entre 2:1 et 10:1;
(iii) incorporer l'extrait antimicrobien dans la base de gel, l'extrait antimicrobien étant incorporé dans une quantité de 1% à 20% en poids de la composition et la base de gel étant incorporée dans une quantité de 60% à 95% en poids de la composition, et à les mélanger ensemble, pour obtenir une composition qui forme une structure cristalline liquide au contact du fluide gingival.

11. Procédé selon la revendication 10, dans lequel l'extrait antimicrobien est incorporé dans une quantité de 1% à 15% en poids de la composition.

12. Procédé selon la revendication 10 ou la revendication 11, dans lequel la composition est préparée sous forme de gel, puce ou pommade à libération prolongée qui est biodégradable.

13. Procédé selon l'une quelconque des revendications 10 à 12, dans lequel le triglycéride consiste en une ou plusieurs huiles végétales choisies parmi l'huile de sésame, l'huile de tournesol, l'huile de soja et l'huile de carthame.

14. Procédé selon l'une quelconque des revendications 10 à 13, dans lequel l'extrait antimicrobien est préparé à partir de la plante *Andrographis paniculata*.

15. Procédé selon l'une quelconque des revendications 10 à 14, comprenant en outre l'incorporation d'acétate de D-α-tocophérol dans une quantité de 0,5% à 5% en poids de la composition, en tant qu'antioxydant.

16. Procédé selon l'une quelconque des revendications 10 à 15, comprenant en outre l'incorporation de glycérine ou de collagène, ou des deux, dans une quantité totale de 0,1% à 5% en poids de la composition.

17. Utilisation d'une composition selon l'une quelconque des revendications 1 à 9 pour la fabrication d'un médicament d'appoint pour le traitement de la parodontite.
